# EUROPEAN PATENT APPLICATION

(11) **EP 4 527 305 A1**
(43) Date of publication of application: **26.03.2025**
(21) Application number: 23198808.0
(22) Date of filing: 21.09.2023
(51) Int. Cl.: A61B 6/12, A61B 34/20

(54) **INSERTION DEVICE POSITIONING USING MEDICAL IMAGING**

(71) Applicant: Koninklijke Philips N.V., 5656 AG Eindhoven (NL)
(72) Inventor: SAALBACH, Axel, Eindhoven (NL); SCHULZ, Heinrich, Eindhoven (NL); SIRAZITDINOV, Ilyas, 5656AG Eindhoven (NL)
(74) Representative: Philips Intellectual Property & Standards

(57) **Abstract**

Medical imaging is commonly employed to verify the positioning of devices such as lines and tubes in the Intensive Care Unit (ICU) and the Emergency Department (ED). Point-to-point measurements can be misleading, e.g. as a result of bended devices or orientation of the image. There is therefore provided a computer-implemented method of providing guidance on insertion device positioning using medical imaging. The method comprises: obtaining input data comprising a medical image (10) of a subject, wherein the medical image depicts at least part of the insertion device (100); processing the medical image to obtain at least one measurement along the length of the insertion device; and based on the obtained at least one measurement, preparing guidance data (208) for output to the user via a user interface. By taking measurements along the length of the device in this way, the methods and systems described herein provide a more accurate approach to insertion device positioning which overcomes the limitations of simple point-to-point measurements.

## Description

### FIELD OF THE INVENTION

The invention relates to methods and systems for insertion device positioning using medical imaging.

### BACKGROUND OF THE INVENTION

X-ray imaging is commonly employed to verify the positioning of devices such as lines and tubes in the Intensive Care Unit (ICU) and the Emergency Department (ED). For endotracheal tubes (ETT), for example, the distance between the tip of the device and the carina bifurcation is assessed: a well-positioned ETT should be placed 5cm (+/-2cm) above the carina. In case of malpositioning, the device must be repositioned based on the image-based measurements. In existing solutions, two different types of measurements are employed. Either the distance is computed as the Euclidean distance between the tip and the carina in the projected X-ray image plane, or only the distance along the main axis of the image is considered. In practice, such point-to-point measurements can be misleading, e.g. as a result of bended devices or orientation of the image.

### SUMMARY OF THE INVENTION

To better address one or more of these concerns, there is provided, in a first aspect of invention, a computer-implemented method of providing guidance on insertion device positioning using medical imaging. The method comprises:
obtaining input data comprising a medical image of a subject, wherein the medical image depicts at least part of the insertion device;
processing the medical image to obtain at least one measurement along the length of the insertion device; and
based on the obtained at least one measurement, preparing guidance data for output to the user via a user interface.

By taking measurements along the length of the device, the methods and systems described herein provide a more accurate approach to insertion device positioning which overcomes the limitations of simple point-to-point measurements.

Processing the medical image may further comprise locating a pathline of the insertion device in the medical image, wherein the at least one measurement is taken along the pathline of the insertion device. The pathline represents the path which the insertion device takes through the subject's body, regardless of whether the path includes straight, curved, or kinked segments. In one example, the pathline comprises a centerline of the insertion device. In other examples, an outline or contour may be used as the pathline. The pathline may be located using image segmentation. Processing the medical image to locate the pathline of the insertion device may thus comprise performing image segmentation to generate a device segmentation mask which localizes the insertion device and from which the pathline is extracted. For example, performing image segmentation may comprise using a (deep-learning-based) segmentation approach to classify pixels as device or non-device and extracting the pathline therefrom. Additionally or alternatively, the pathline may be located using model-based device detection. For example, locating the pathline may comprise fitting a device model to the medical image (using e.g. an active shape model) from which the pathline is extracted. Additionally or alternatively, locating the pathline may comprise using a search algorithm (for example, an algorithm which follows the insertion device from a starting point). Additionally or alternatively, locating the pathline may comprise detecting control points and connecting the control points to form the pathline, e.g., the centerline. Control points may be detected using a deep learning algorithm, for example, as described in I. Sirazitdinov et al. Bi-directional Encoding for Explicit Centerline Segmentation by Fully-Convolutional Networks. Miccai 2022. It will furthermore be understood that combinations of pathline localization techniques may also be used.

Measurements along the length of the insertion device may then be taken in any appropriate way, for example using any appropriate image analysis technique. By "measurement along the length of the insertion device" is meant herein any measurement taken along the pathline of the insertion device, i.e., one which takes into account any straight, curved, or kinked part of the pathline. The length which is measured may thus be described as the rectified length of the segment being measured: that is, the length which the segment would exhibit if it were rectified to give a straight line segment. This does not imply that the segment must in fact be rectified for it to exhibit a rectified length. This is in contrast to the Euclidean distance which is conventionally measured between the start- and endpoints of the segment. In one example, the at least one measurement along the length of the insertion device is obtained via interpolation along the set of control points representing the pathline. For example, the measurement along the length of the insertion device may be taken by interpolating between n-last control points (to take into account the curvature of the insertion device). Linear interpolation results in straight line segments, whereas spline interpolation of order > 1 results in smooth curves. The measurement is calculated as the length between the start- and endpoints over the interpolated path. In another example, the at least one measurement along the length of the insertion device is obtained by accumulating distances per pixel along the pathline in the device segmentation mask.

The measurement may be taken of the length of a segment of the insertion device defined by start- and endpoints. The start- and/or endpoint may be defined by a landmark such as anatomical landmark, a predetermined point on the insertion device, or a target region for the insertion device. The method may further comprise obtaining data defining the target region for the insertion device, for example a user definition of the target region. Processing the medical image to obtain the at least one measurement along the length of the insertion device may comprise computing a displacement between any two landmarks, such as at least one predetermined point on the insertion device and the target region. The displacement again accounts for a straight, curved, or kinked pathline. The at least one predetermined point may comprise a tip of the insertion device.

The measured length may then be output to the user as part of the guidance data on device repositioning. Processing the medical image may further comprise calculating a correction to the position of the insertion device which, when implemented, causes the at least one predetermined point on the insertion device to reach another landmark such as the target region (for example by reaching a boundary or center of the target region), wherein the calculated correction comprises an adjustment to an inserted length of the insertion device. The correction may be calculated on the basis of the at least one measurement along the length of the device, such as the displacement between landmarks. Calculating the adjustment to the inserted length of the insertion device may comprise model-based prediction of an effect of the adjustment when performed on the pathline of the insertion device. Guidance data which is provided to the user in the form of a length adjustment in this way is readily translatable into an action required to reposition the device correctly, particularly in the case of insertion devices such as tubes, lines or catheters, which may be repositioned by pushing or pulling the device, that is, by inserting or retracting the device. To provide readily actionable guidance to the user, the calculated correction may comprise an indication of whether to push or pull the insertion device to reach the target region, and/or an indication of how far to push or pull the insertion device to reach the target region.

Generally, the guidance data may comprise one or more of: the obtained at least one measurement; the computed displacement between the at least one predetermined point on the insertion device and the target region; and the calculated correction to the position of the insertion device for output to the user via the user interface. Preparing the guidance data for output to the user via the user interface may comprise generating a graphical illustration depicting the position of the insertion device relative to the target region. The graphical illustration may further depict any of the guidance data described herein, such as the obtained at least one measurement, and/or the computed displacement between the at least one predetermined point on the insertion device and the target region, and/or the calculated correction to the position of the insertion device. The graphical illustration may depict relevant information by highlighting or annotating parts of the medical image, so as to provide intuitive guidance to the user on device repositioning.

The methods and systems described herein are widely applicable in the field of device positioning using medical imaging. The medical image may comprise an X-ray image, for example. According to a second aspect, there is provided a computing system configured to perform the method of the first aspect.

According to a third aspect, there is provided a computer program (product) comprising instructions which, when executed by a computing system, enable or cause the computing system to perform the method of the first aspect.

According to a fourth aspect, there is provided a computer-readable (storage) medium comprising instructions which, when executed by a computing system, enable or cause the computing system to perform the method of the first aspect. The computer-readable medium may be transitory or non-transitory, volatile or non-volatile.

By "insertion device" is meant any line, tube, catheter, or other flexible device which is insertable into a patient and detectable using medical imaging. In some particular examples, the insertion device may comprise an endotracheal tube (ETT), a central venous catheter (CVC), or a nasogastric tube (NGT). In those cases, the target region may comprise the carina of the subject for the endotracheal tube, the superior vena cava for the central venous catheter, or the stomach for the nasogastric tube.

The "subject" of the medical imaging may be human or animal.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:
Figs. 1A and 1B illustrate examples of medical images depicting malpositioned ETTs;
Fig. 2 schematically illustrates an algorithm for providing guidance on insertion device positioning using medical imaging;
Figs. 3A and 3B illustrates examples of guidance data which may be provided by the algorithm of FIG. 2; and
Fig. 4 illustrates a computing system that can be used in accordance with the systems and methods disclosed herein.

The X-ray images depicted in the figures are taken with permission from the database provided by the NIH Clinical Center, available at https://nihcc.app.box.com/v/ChestXray-NIHCC, and described in Wang X, Peng Y, Lu L, Lu Z, Bagheri M, Summers RM. ChestX-ray8: Hospital-scale Chest X-ray Database and Benchmarks on Weakly-Supervised Classification and Localization of Common Thorax Diseases. IEEE CVPR 2017.

### DETAILED DESCRIPTION OF EMBODIMENTS

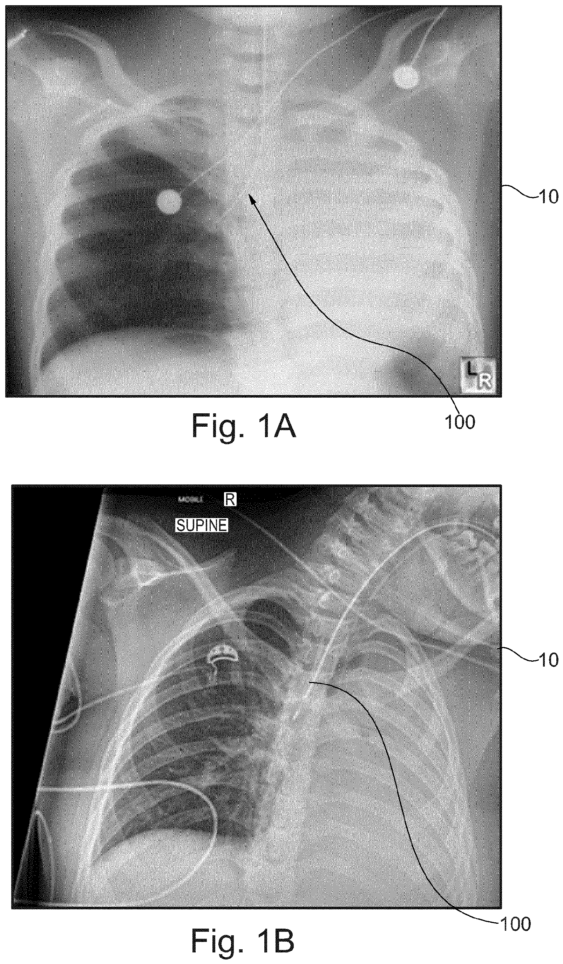

Fig. 1A shows an X-ray image 10 with a strongly curved endotracheal tube (ETT) 100 in one of the bronchi. In such circumstances, point-to-point based distance measurements will tend to underestimate the inserted length due to the bending of the tube 100 in the bronchi. Furthermore, conventional measurement techniques will only show the distance to the carina and not the displacement to the tube 100 which is required to bring it into the proper position.

Fig. 1B shows another X-ray image 10, which is rotated, again with a malpositioned ETT 100. The rotation of the image introduces additional measurement errors if only the main axis of the image is considered.

Measurements derived from the X-ray image are essential for the assessment of the positioning of the insertion device 100 and for the determination of corrective actions. To provide more meaningful measurements and visualizations, the present disclosure proposes an algorithm for providing guidance on insertion device positioning using device-based measurements taken from the medical image 10. In contrast to point-to-point measurements, the approach disclosed herein takes measurements along the length of the insertion device 100 using image processing techniques.

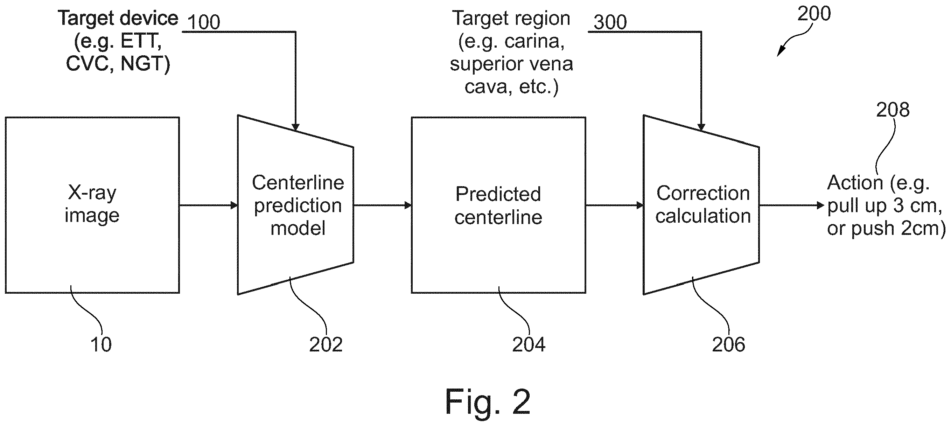

Fig. 2 schematically illustrates the algorithm 200 for providing guidance on insertion device positioning using medical imaging.

The algorithm 200 receives, as input, a medical image such as an X-ray image 10. A centerline prediction model 202 processes the X-ray image 10 together with input data indicating the type of insertion device 100 ("target device") being used, e.g., an ETT, CVC, or NGT, to output a predicted centerline 204 of the insertion device 100.

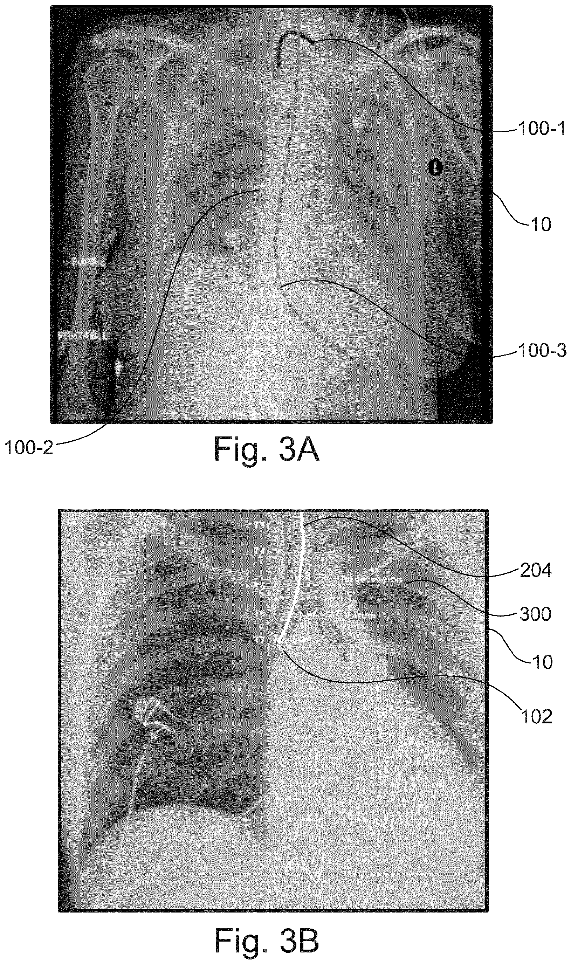

Fig. 3A shows an X-ray image 10 annotated to illustrate the result of the application of the centerline prediction model 202. The centerline prediction model 202 extracts the centerline 204 of the insertion device 100 as a set of control points. The centerline 204 may be extracted for example using the approach described in I. Sirazitdinov et al. Bi-directional Encoding for Explicit Centerline Segmentation by Fully-Convolutional Networks. Miccai 2022. Fig. 3A illustrates centerlines extracted as sets of control points for various insertion devices 100 including an ETT 100-1, a CVC 100-2, and an NGT 100-3. Measurements along the predicted centerline 204 are obtained via interpolation along the set of control points.

Returning to Fig. 2, the predicted centerline 204 is input to a correction calculation model 206, which additionally receives, as input, data defining a target region 300 for the insertion device 100. The target region 300 may be user defined. For example, the target region 300 may comprise the carina for an ETT, the superior vena cava for an CVC, or the stomach for an NGT. The correction calculation model 206 calculates a corrective action 208 that is required for the insertion device 100 to reach the target region 300, e.g. the ETT must be pulled up by 3 cm, or pushed by 2 cm.

The algorithm 200 concludes with the preparation of guidance data for output to the user via a user interface. For example, the guidance data may be used to inform radiologists and/or ICU/ED staff about the positioning of the insertion device, optionally with the related measurements. The user interface may form part of an X-ray imaging system, for example (for early detection of misplaced devices) and/or part of a PACS viewer or a diagnostic workstation.

Fig. 3B illustrates guidance data which may be output to the user via the user interface. In this example, the guidance data comprises an X-ray image 10 which is annotated to indicate the extracted centerline 204 of the ETT 100 along with measurements taken along the length along the insertion device 100 between specific landmarks (shown here as including the displacement between the tip 102 of the ETT 100 and the carina, and the displacement between the tip 102 and the center of the target region 30). The guidance data provides an indication of corrective actions that are required to reposition the ETT: retracting the ETT by 3cm brings the tip to the level of the carina whereas, to position the ETT according to the target region specified by clinical guidelines, an 8cm retraction is required.

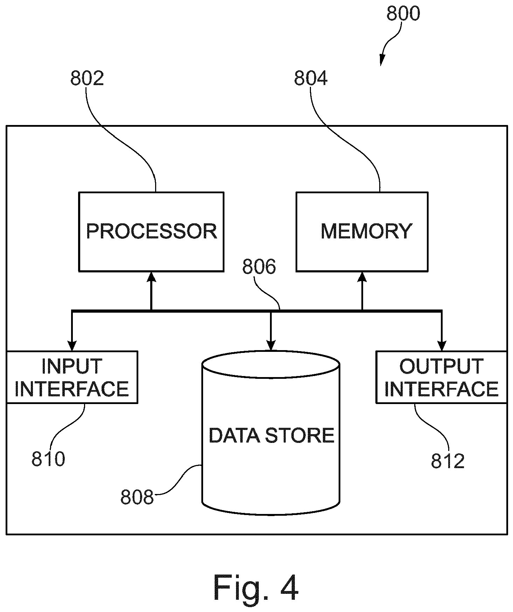

Fig. 4 illustrates an exemplary computing system 800 that can be used in accordance with the systems and methods disclosed herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by one or more components described herein or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. A computer-implemented method of providing guidance on insertion device positioning using medical imaging, the method comprising:
obtaining input data comprising a medical image (10) of a subject, wherein the medical image depicts at least part of the insertion device (100);
processing the medical image to obtain at least one measurement along the length of the insertion device; and
based on the obtained at least one measurement, preparing guidance data (208) on insertion device positioning for output to the user via a user interface.

2. The method of claim 1, wherein processing the medical image further comprises locating a pathline (204) of the insertion device (100) in the medical image (10), wherein the at least one measurement is taken along the pathline of the insertion device.

3. The method of claim 2, wherein the pathline comprises a centerline (204) of the insertion device (100).

4. The method of claim 2 or 3, wherein processing the medical image (10) to locate the pathline (204) of the insertion device (100) comprises performing image segmentation to generate a device segmentation mask which localizes the insertion device, and wherein processing the medical image to obtain the at least one measurement along the length of the insertion device comprises accumulating distances per pixel in the device segmentation mask.

5. The method of any preceding claim, further comprising obtaining a user definition of a target region (300) for the insertion device (100); wherein processing the medical image (10) to obtain the at least one measurement along the length of the insertion device comprises computing a displacement between at least one predetermined point on the insertion device and the target region.

6. The method of claim 4, wherein processing the medical image further comprises calculating a correction (208) to the position of the insertion device (100) which, when implemented, causes the at least one predetermined point on the insertion device to reach the target region (300), wherein the calculated correction comprises an adjustment to an inserted length of the insertion device.

7. The method of claim 6, wherein calculating the adjustment (208) to the inserted length of the insertion device (100) comprises model-based prediction of an effect of the adjustment when performed on the path of the insertion device.

8. The method of claim 6 or 7, wherein the calculated correction (208) comprises an indication of whether to push or pull the insertion device (100) to reach the target region (300).

9. The method of claim 8, wherein the calculated correction (208) further comprises an indication of how far to push or pull the insertion device (100) to reach the target region (300).

10. The method of any preceding claim, wherein preparing the guidance data for output to the user via the user interface comprises generating a graphical illustration depicting the position of the insertion device (100) relative to a target region (300).

11. The method of claims 6 and 10, wherein the graphical illustration further depicts the adjustment to the inserted length of the insertion device (100) to reach the target region (300).

12. The method of claim 10 or 11, wherein the graphical illustration depicts relevant information by highlighting or annotating parts of the medical image (10).

13. The method of any preceding claim, wherein the insertion device (100) comprises an endotracheal tube, a central venous catheter, or a nasogastric tube, and wherein a target region (300) comprises the carina of the subject for the endotracheal tube, the superior vena cava for the central venous catheter, or the stomach for the nasogastric tube.

14. A computing system (800) configured to perform the method of any preceding claim.

15. A computer-readable medium (804; 808) comprising instructions which, when executed by a computing system (800), cause the computing system to perform the method of any of claims 1-13.
